# EUROPEAN PATENT APPLICATION

(11) **EP 4 686 454 A1**
(43) Date of publication of application: **04.02.2026**
(21) Application number: 25192553.3
(22) Date of filing: 29.07.2025
(51) Int. Cl.: A61B 17/72

(54) **INTRAMEDULLARY NAIL**

(30) Priority: 01.08.2024 US 202463678213 P; 28.07.2025 US 202519282006
(71) Applicant: Stryker European Operations Limited, Carrigtwohill T45H X08 (IE)
(72) Inventor: Kleiner, Oliver, Schwentinental (DE); Gerber, Claus, Heikendorf (DE); Zander, Nils, Eckernförde (DE)
(74) Representative: Maiwald GmbH

(57) **Abstract**

An intramedullary nail includes a cylindrical portion, a non-cylindrical portion, and a screw hole. The cylindrical portion includes a longitudinal axis and a diameter. The non-cylindrical portion includes a first dimension equal to the diameter and a second dimension greater than the diameter. The first and the second dimensions are perpendicular to the longitudinal axis. The screw hole extends through the non-cylindrical portion in a direction transverse to the longitudinal axis.

## Description

### CROSS-REFERENCE TO RELATED APPLICATIONS

This application claims the benefit of the filing date of United States Provisional Patent Application No. 63/678,213 filed on August 1, 2024.

### BACKGROUND OF THE INVENTION

Intramedullary nails are commonly used for fixation of long bone fractures in various areas of the body. Intramedullary nails are often advantageous in comparison to extra-medullary methods, as intramedullary nails provide reliable and long-lasting stabilization and are associated with low complications due to implant fatigue breakages. Thus, intramedullary nails widely considered a standard treatment to stabilize long bone fractures until bony consolidation occurs.

However, in cases of complex fracture patterns, unfavorable healing preconditions (*e.g.,* comorbidities and risk factors for delayed healing), or improper placement or locking of a nail, fatigue may occur in the nail causing subsequent failure. Currently, most nails have a cylindrical cross-section and are designed to not only cooperate with anatomical structures, but also existing prosthetics such as femoral implants. Where typical engineering principals would dictate increasing the overall cross-sectional size of the implant to increase strength of the nail, the shape and dimensions of the anatomical structures and existing components limit this possibility.

Thus, there is a need for an intramedullary nail having increased strength that is able to withstand fatigue while maintaining universal use and ease of insertion.

### BRIEF SUMMARY OF THE INVENTION

The present disclosure addresses the aforementioned drawbacks by providing an intramedullary nail having an increased strength to prevent fatigue and nail failure, the nail having a limited material agglomeration at critical areas of the nail around screw holes. The limited material agglomeration results in a partial circumference increase of a cross-section of the nail which increases the fatigue strength of the nail.

In accordance with an aspect of the present disclosure, an intramedullary nail includes a cylindrical portion, a non-cylindrical portion, and a screw hole. The cylindrical portion includes a longitudinal axis and a diameter. The non-cylindrical portion includes a first dimension equal to the diameter and a second dimension greater than the diameter, the first and the second dimensions are perpendicular to the longitudinal axis. The screw hole extends through the non-cylindrical portion in a direction transverse to the longitudinal axis.

Embodiments of this first aspect may include a protrusion on each end of the second dimension of the non-cylindrical portion.

In a further aspect, the second dimension may extend in a direction transverse to the first dimension.

In some aspects, an edge of the protrusion may be straight. In other aspects, an edge of the protrusion may be curved.

Embodiments of this first aspect may include a third dimension of the non-cylindrical portion equal to the second dimension of the non-cylindrical portion, the second and the third dimensions having a protrusion on each end of the second and the third dimensions.

In a further aspect, the third dimension may extend in a direction transverse to the first and the second dimensions.

In yet a further aspect, an edge of the protrusion may be curved.

In yet a further aspect, the screw hole may include a plurality of screw holes. A periphery of the screw hole may be aligned with the second dimension.

In yet a further aspect, the non-cylindrical portion may have an increased strength over the cylindrical portion.

In yet a further aspect, the nail may further include a hole extending along the longitudinal axis at a center point of the first and the second dimensions.

In yet a further aspect, the non-cylindrical portion may be subjected to increased stress when the screw hole of the non-cylindrical portion is empty.

In yet a further aspect, the nail may further include a plurality of non-cylindrical portions.

In accordance with another aspect of the present disclosure, an intramedullary nail includes a cylindrical body having a diameter, a transverse bore extending through the cylindrical body, and a protrusion aligned with the transverse bore. A cross-section extending through the protrusion includes a first dimension equal to the diameter and a second dimension greater than the diameter.

Embodiments of this second aspect may include a first protrusion on a first end of the second dimension and a second protrusion symmetrical about a longitudinal axis of the intramedullary nail on a second end of the second dimension. The protrusion may be a first protrusion.

In some aspects, the first and the second protrusions may have a straight edge with curved corners that taper inward towards the cylindrical body. In other aspects, the first and the second protrusions may have a curved edge.

Embodiments of this second aspect may include a third dimension equal to the second dimension, the third dimension extends transverse to the first and second dimensions. The protrusion may be a first protrusion on a first end of the second dimension, and the intramedullary nail may further include a second protrusion symmetrical about a longitudinal axis of the intramedullary nail on a second end of the second dimension.

In a further aspect, the third dimension may include a third and fourth protrusion on a first end and a second end of the third dimension. The third protrusion may be symmetrical about a longitudinal axis of the intramedullary nail to the fourth protrusion.

In yet a further aspect, the first, the second, the third, and the fourth protrusions each may have a curved edge.

### BRIEF DESCRIPTION OF THE DRAWINGS

A more complete appreciation of the subject matter of the present disclosure and of the various advantages thereof can be realized by reference to the following detailed description in which reference is made to the accompanying drawings in which:
Fig. 1 is a perspective view of an intramedullary nail according to an embodiment of the present disclosure.
Fig. 2 is a cross-sectional view of the intramedullary nail of FIG. 1 taken along Line 2-2.
Fig. 3 is a cross-sectional view of an intramedullary nail according to another embodiment of the present disclosure.
Fig. 4 is a cross-sectional view of an intramedullary nail according to another embodiment of the present disclosure.
Fig. 5 is a schematic diagram illustrating an increase strength of an intramedullary nail according to the present disclosure in comparison with a conventional intramedullary nail.

### DETAILED DESCRIPTION

In describing the preferred embodiments of the invention, specific terminology will be used for the sake of clarity. However, the invention is not intended to be limited to any specific terms used herein, and it is to be understood that each specific term includes all technical equivalents, which operate in a similar manner to accomplish a similar purpose. In the drawings and in the description which follows, the term "proximal" means closer to the heart and the term "distal" means more distant from the heart. The term "anterior" means towards the front part of the body or the face and the term "posterior" means towards the back of the body. The term "medial" means toward the midline of the body and the term "lateral" means away from the midline of the body.

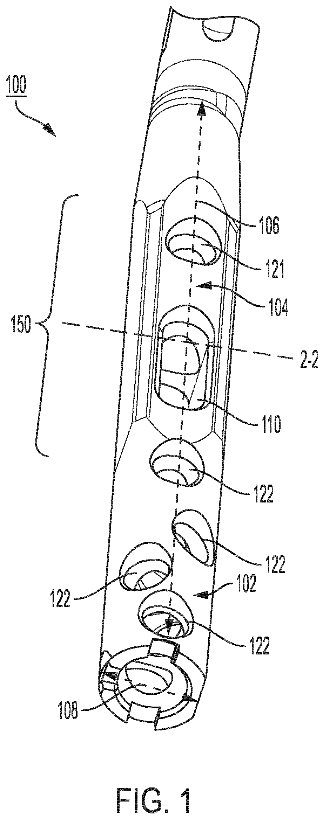

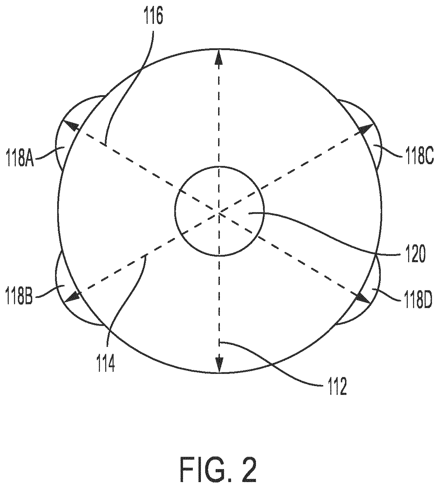

In accordance with one aspect of the present disclosure, an intramedullary nail 100 includes a cylindrical portion 102, a non-cylindrical portion 104, and a screw hole 110. Referring to Fig. 1, cylindrical portion 102 includes a longitudinal axis 106 and a diameter 108. Longitudinal axis 106 extends across a length of nail 100 through a center point of a circumference of a cross-section of nail 100. Diameter 108 extends from end to end of an outer circumference of a cross-section of cylindrical portion 102 of nail 100. As shown in Fig. 2, non-cylindrical portion 104 includes a first dimension 112 equal to diameter 108 of cylindrical portion 102 and second and third dimensions 114, 116 that are both greater than diameter 108. First and second dimensions 112, 114 are perpendicular to longitudinal axis 106 and extend across a cross-section of non-cylindrical portion 104 of nail 100.

As shown in Fig. 1, screw hole 110 extends through non-cylindrical portion 104 in a direction transverse to longitudinal axis 106, defining a critical area 150 along nail 100. In some examples, in the direction transverse may be defined as a direction perpendicular or otherwise a direction at an angle. In the embodiment shown, nail 100 includes holes 110, 121 within critical area 150 along the nail. Screw hole 110 is a compression hole, whereas hole 121 is a circular fixation hole. Nail 100 also includes one or more fixation holes 122 located outside of critical area 150. In other embodiments, any number of holes or types of holes may be included in a given critical area, or in the entirety of the nail. These holes may be locking holes or any other type of hole. For instance, screw hole 110 may be an elongated or oblong hole configured to receive a screw, controlling apposition and compression to the fracture site. As shown in Fig. 1, fixation holes 122 are offset from longitudinal axis 106 extending through the center point of the circumference of nail 100. Compression hole 110 and circular fixation hole 121 are aligned along longitudinal axis 106 of nail 100. In other embodiments, the fixation holes and compression holes may be offset from the longitudinal axis or aligned with the longitudinal axis of the nail in any combination.

Typically, compression hole 110 and circular fixation hole 121 are only engaged by a screw for treatment of simple shaft failures. In some embodiments, such as clinical fracture situations, compression hole 110 and circular fixation hole 121 remain empty. In other embodiments, such as complex distal femur fractures, compression hole 110 and circular fixation hole 121 remain non-locked or empty. Locking or filling the holes with screws may decrease the stress inside the nail along the respective area of fixation holes 122. As such, compression hole 110 and circular fixation hole 121 have an increased risk of fatigue failure over more distal holes such as fixation holes 122 that are more likely to be locked or filled with screws. In other embodiments, critical area 150 may include areas of the nail having fixation holes 122 depending on the type of fracture treated by the nail.

As shown in Fig. 1 and Fig. 2, a periphery of screw hole 110 is aligned with second dimension 114. Each critical area 150 includes non-cylindrical portion 104 having first dimension 112 equal to diameter 108 of cylindrical portion 102 of nail 100 along a non-critical area and second dimension 114 having a length greater than first dimension. The areas of material agglomeration are located around critical areas of the nail having a screw hole. Critical high stress areas can be determined around screw holes in highly predictable locations based off data of typical areas of failure and fatigue in nails.

In some embodiments, the intramedullary nail may include a proximal section, a distal section, and an intermediate section positioned between the proximal and distal sections. The proximal and distal section may be substantially straight while the intermediate section includes a curved portion. The nail may include a plurality of screw holes extending through each of the proximal section, the intermediate section, and the distal section. The proximal section, the intermediate section, and the distal section may include a combination of compression holes and fixation holes. In some embodiments, the nail may also include static screw holes, transverse screw holes, and recon screw holes. In some embodiments, the nail may only include holes along a proximal portion of the nail or only a distal portion of the nail. An example of an intramedullary nail is disclosed in U.S. Pat. No. 11,013,540, the disclosure of which is incorporated herein by reference in its entirety. As shown in Figs. 1 and 2, nail 100 is hollow and includes a hole 120 extending along longitudinal axis. Hole 120 extends along the longitudinal axis at a center point of first and second dimensions 112, 114. In some embodiments, a portion of nail 100 may be threaded along a portion of hole 120 and to mate with a cap. In other embodiments, the nail may be an intermediate femoral nail as disclosed in U.S. Pat. No. 18,387,290, the disclosure of which is incorporated herein by reference in its entirety.

Referring to Fig. 2, the cross-section along Line 2-2 of non-cylindrical portion 104 of nail 100 of Fig. 1 is shown in further detail. As shown, non-cylindrical portion 104 includes first dimension 112 equal to that of diameter 108, as well as second and third dimensions 114, 116 having a length that is equal to each other, but larger than that of first dimension 112. Essentially, second and third dimensions 114, 116 are created by the inclusion of protrusions 118A-118D on each end of second and third dimensions. Second and third dimensions 114, 116 extend in directions transverse to first and second dimensions 112, 114, and across non-cylindrical portion 104 of nail 100.

Each protrusion 118A-118D is rounded, thereby resulting in a cloverleaf shape at the cross-section. This results in a limited material agglomeration around the critical area of nail having screw hole 110. Although shown rounded the protrusion can exhibit many different shaped, for instance, exhibiting a straight edge or multiple edges forming a rectangular or polygonal shape. Moreover, although four protrusions are shown, any number may be included. It is to be understood that even small amounts of material agglomeration may increase the strength of the nail, and as such, the material agglomeration may be partial in circumference and length. Protrusions 118A-118D result in a partial circumference increase of then nail without completely increasing the cross-section circumferentially, and thus, not impacting insertion characteristics of the nail.

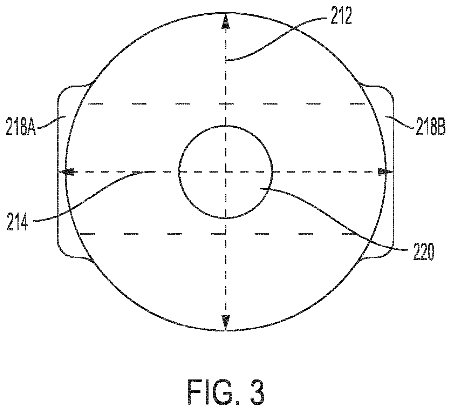

An example of a different embodiment cross-sectional shape for a critical area is shown in Fig. 3. There, a non-cylindrical portion of a nail 200 (not shown) includes protrusions 218A, 218B on each end of a second dimension 214. Nail 200 is similar to that of nail 100, except in regard to the differences in the cross-section of the non-cylindrical portion as described below. Second dimension 214 extends in a direction transverse to a first dimension 212. Hole 220 is positioned at a center point of first and second dimensions 212, 214. Each protrusion 218A, 218B extends from one side of the cross-section of nail 200, resulting in a greater second dimension 214 length over first dimension 212. As shown in FIG. 3, protrusions 218A, 218B have straight edges, defining a rectangular protrusion extending from the outer surface of the non-cylindrical portion of nail 200. In other embodiments, each protrusion 218A, 218B may exhibit different designs, including a straight edge with curved corners that taper inwards towards the outer surface of nail 200.

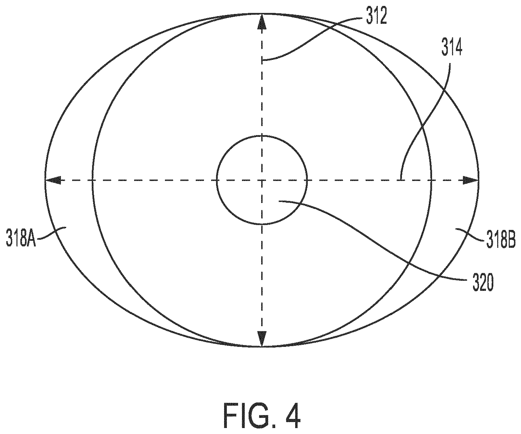

Another embodiment is shown in FIG. 4. There, a non-cylindrical portion of a nail 300 (not shown) includes protrusions 318A, 318B. Nail 300 is similar to that of nails, 100, 200, except in regard to the differences in the protrusions as described below. Second dimension 314 extends in a direction transverse to a first dimension 312. Hole 320 is positioned at a center point of first and second dimensions 312, 314. Protrusions 318A, 318B have a curved edge that cooperates with other portions of the nail to result in an oval cross-section.

While certain embodiments of material agglomeration are provided in the figures, the present disclosure is not limited as such and the protrusions may be of various shapes, sizes, or quantities. In some embodiments, the cross-section may include rounded protrusions, protrusions with pointed edges, or polygonal shaped protrusions. The protrusions may be symmetrical about an axis extending end to end across the cross-section. In other embodiments, the protrusions may be asymmetrical about the axis. The protrusions may be of different sizes along the cross-section of the nail.

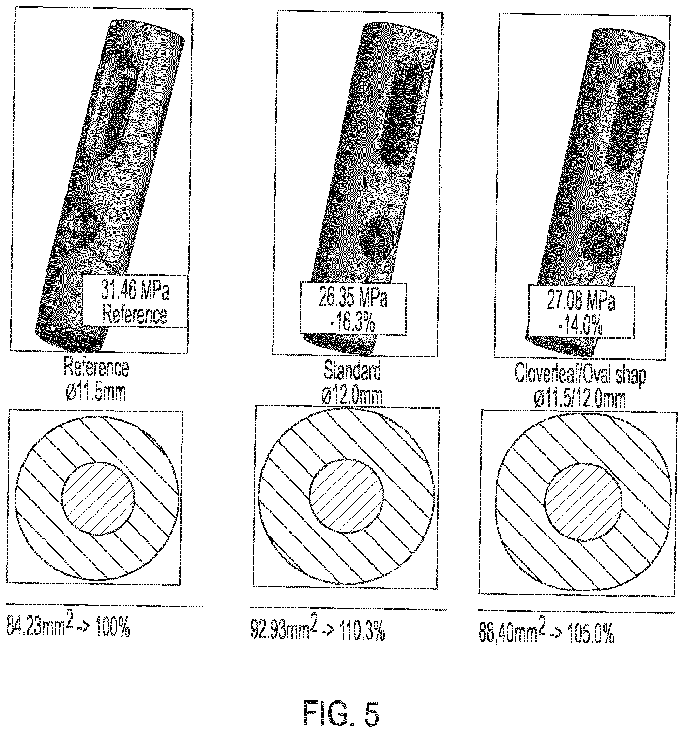

As shown in Fig. 5, the non-cylindrical portion of the nail has an increased strength over the cylindrical portion of the nail. Conventionally, a uniform increase in the cylindrical diameter of a nail results to about a 16% strength improvement. For example, a slight increase in the outer diameter of a cylindrical nail from a diameter of 11.5 mm to 12 mm leads to a 16% strength improvement with an increased overall circumference. However, examples of limited material agglomeration as taught in the present disclosure may also result in a comparable strength increase. Data determined from FEAs calculating relative stress levels of the cloverleaf cross-section shows a 14% strength improvement with a 5% the size of the cross-section. As such, increased fatigue strength with limited impact on insertion characteristics is achieved using the limited material agglomeration. In other words, local material agglomeration around the critical areas of the nail can be used to achieve strength increase without extending the lateral width and thus allowing for unhindered insertion.

For example, in the case of the cloverleaf shaped cross-section as shown in Fig. 2, the diameter or first dimension of the non-cylindrical portion of the nail may remain as 11.5 mm or equal to the diameter of the cylindrical portion of the nail. The second and third dimensions of the non-cylindrical portion of the nail may be increased to 12 mm, resulting in a 14 % strength increase with limited material agglomeration along two dimensions.

In some embodiments, the varying cross-sections along the intramedullary nail may allow for a torque resisting cross-section restricted to certain areas of the implant. In other embodiments, the cross-section and shape could be designed to optimally conform to the inner contour of the bone such as distal fibula nailing.

While certain embodiments of the cross-section of the nail disclosed herein are shown in the accompany drawings, it is to be understood that additional shapes and designs for the cross-section could be designed for the nail. For instance, the cross-section may include any polygonal shape with limited material agglomeration and not a full increase of the circumference of the nail.

In accordance with yet another aspect of the present disclosure, a method of inserting an intramedullary nail includes inserting an intramedullary nail into a medullary canal of a long bone such as the femur or the tibia. In some examples, a reamer is used in combination with a K-wire to open the medullary canal to prepare the canal for insertion of the nail. The reamer may include an opening reamer handle and an opening reamer. A cortex of the medullary canal is first penetrated by the K-wire. Then, the reamer is used to access the medullary canal and create an opening for the nail. In other examples, the medullary canal may be opened with a curved awl. In some instances, the intramedullary nail may be inserted without reaming in patients with wide medullary canals.

After the opening in the medullary canal is prepared, the intramedullary nail is inserted into the canal using a targeting arm. Prior to insertion, the alignment of the nail is verified by inserting a drill through a sleeve and targeting arm assembly. The targeting arm assembly may include a nail holding screw. In some examples, the selected nail is assembled on a nail adapter with a nail holding screw having an insertion wrench, the nail adapter is then attached to a targeting arm. In instances where recon is desired, a recon knob may be attached to the targeting arm. The alignment and depth of nail insertion may vary depending on each particular case. Once the nail is inserted within the canal, screws are inserted transverse through the bone into the nail to fix the nail in place. Screw trajectory through the bone and into the screw hole of the nail is verified prior to insertion of each screw.

In cases having insertion restrictions such as restrictions on the nail dimension in the medial lateral direction due to the presence of a femoral implant, insertion implement or other component, the nail of the present disclosure can be inserted with ease. Unlike a nail having a full circumference diameter increase, limited material agglomeration along a critical area of the nail only increases the diameter of the nail along one or more dimensions not the entire circumference of the nail. As such, the nail can still be inserted within restrained openings in femoral implants or other components. For example, in cases of retrograde femur nail insertion through a TKA box in peri prosthetic indications, the femur may have an entry portal restriction in the medial lateral direction requiring a specific nail diameter for insertion. The nail of the present disclosure does not require a larger sized hole in the canal as the femur has elastic properties which allow for insertion of the nail using a hammer. The limited material agglomeration only requires limited bone deformation which may be achieved from the elastic properties of the femur.

In accordance with yet another aspect of the present disclosure a method of manufacturing an intramedullary nail includes using a cutting tool to remove material from the outer diameter of a rotating workpiece to form a polygonal or oval shaped cross-section of a nail. In some embodiments, the cross-sections having limited material agglomeration may be manufactured using turning equipment. Additionally, the intramedullary nail may be made of a biocompatible material such as a polymer e.g., reinforced polymer composites; a titanium alloy e.g., Ti6Al4V; stainless steel e.g., 316L; or other metals and metal alloys, such as Ti, StSt, and CoCr. In other embodiments, a material having a high fatigue strength may be used for the entire nail.

Although the invention herein has been described with reference to particular embodiments, it is to be understood that these embodiments are merely illustrative of the principles and applications of the present disclosure. For example, features described in relation to one particular embodiment may be combined with features of other embodiments described herein. It is therefore to be understood that numerous modifications may be made to the illustrative embodiments and that other arrangements may be devised without departing from the scope of the present disclosure as defined in the appended claims.

## Claims

1. An intramedullary nail, comprising:
a cylindrical portion (102) having a longitudinal axis (106) and a diameter (108);
a non-cylindrical portion (104) having a first dimension (112) equal to the diameter (108) and a second dimension (114) greater than the diameter (108), the first and second dimensions (112, 114) being perpendicular to the longitudinal axis (106); and
a screw hole (110) extending through the non-cylindrical portion (104) in a direction transverse to the longitudinal axis (106).

2. The intramedullary nail of claim 1, wherein the non-cylindrical portion (104) includes a protrusion (118B, 118C) on each end of the second dimension (114).

3. The intramedullary nail of claim 1 or claim 2, wherein the second dimension (114) extends in a direction transverse to the first dimension (112).

4. The intramedullary nail of claim 2 or claim 3, wherein an edge of the protrusion (218A, 218B) is straight.

5. The intramedullary nail of claim 2 or claim 3, wherein an edge of the protrusion (118B, 118C, 318A, 318B) is curved.

6. The intramedullary nail of any one of claims 1-5, wherein the non-cylindrical portion (104) includes a third dimension (116) equal to the second dimension (114), the second and the third dimensions (114, 116) having a protrusion (118B, 118C, 118A, 118D) on each end of the second and the third dimensions (114, 116).

7. The intramedullary nail of claim 6, wherein the third dimension (116) extends in a direction transverse to the first and the second dimensions (112, 114).

8. The intramedullary nail of claim 6 or claim 7, wherein an edge of the protrusion (118B, 118C, 118A, 118D) is curved.

9. The intramedullary nail of any one of claims 1-8, wherein the screw hole (110) includes a plurality of screw holes.

10. The intramedullary nail of any one of claims 1-9, wherein a periphery of the screw hole (110) is aligned with the second dimension (114).

11. The intramedullary nail of any one of claims 1-10, wherein the non-cylindrical portion (104) has increased strength compared to the cylindrical portion (102).

12. The intramedullary nail of any one of claims 1-11, further comprising a hole (120) extending along the longitudinal axis (106) at a center point of the first and the second dimensions (112, 114).

13. The intramedullary nail of any one of claims 1-12, wherein the non-cylindrical portion (104) is subjected to increased stress when the screw hole (110) of the non-cylindrical portion is empty (104) and the nail is subject to a load.

14. The intramedullary nail of any one of claims 1-13, further comprising a plurality of non-cylindrical portions.
